# EUROPEAN PATENT SPECIFICATION

(11) **EP 4 267 056 B1**
(45) Date of publication and mention of the grant of the patent: **28.01.2026**
(21) Application number: 21824050.5
(22) Date of filing: 03.12.2021
(51) Int. Cl.: A61F 9/007

(54) **PROXIMAL BYPASS CHANNEL**
PROXIMALER BYPASS-KANAL
CANAL DE DÉRIVATION PROXIMAL

(30) Priority: 22.12.2020 US 202017130409; 21.07.2021 US 202117381980
(43) Date of publication of application: 01.11.2023
(73) Proprietor: Johnson & Johnson Surgical Vision, Inc., Irvine, CA 92618 (US)
(72) Inventor: GOVARI, Assaf, 2066717 Yokneam (IL); ALGAWI, Yehuda, 2066717 Yokneam (IL); AHARON, Eran, 2066717 Yokneam (IL); SITNITSKY, Ilya, 2066717 Yokneam (IL); KATZIR, Stanislav, 2066717 Yokneam (IL)
(74) Representative: Carpmaels & Ransford LLP
(86) International application number: PCT/IB2021/061317
(87) International publication number: WO 2022/136987

(56) References cited:
- WO-A1-2009/076717
- RU-C1- 2 720 821
- US-A1- 2005 118 048
- US-A1- 2008 319 374
- US-A1- 2010 030 134
- US-A1- 2014 163 455

## Description

### FIELD OF THE INVENTION

The present invention relates to medical systems, and in particular, but not exclusively to, fluid dynamics in medical systems.

### BACKGROUND

A cataract is a clouding and hardening of the eye's natural lens, a structure which is positioned behind the cornea, iris and pupil. The lens is mostly made up of water and proteins and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution (BSS) to maintain the anterior chamber of the eye. After removing the cataract with phacoemulsification, the softer outer lens cortex is removed with suction. An intraocular lens (IOL) is then introduced into the empty lens capsule restoring the patient's vision.

The disclosure of US2014/163455A1 provides a method that includes directing a fluid through an aspiration conduit in a phacoemulsification hand piece using a vacuum pressure created from a pump in the hand piece interfacing with the aspiration conduit and directing an irrigation fluid through an irrigation conduit in the hand piece using a pressure created from the pump interfacing with the irrigation conduit. The method also includes increasing an irrigation fluid flow through the irrigation conduit by activating the pump in the hand piece, detecting a pressure associated with a surgical site using a sensor, and controlling intraocular pressure (IOP) by adjusting the pump speed based on the detected pressure.

The disclosure of RU2720821C1 provides an irrigation system that consists of interconnected series of pre-cassette hose, replaceable cassette with channel and distributing rotary valve, post-cassette hose, irrigation channel of cannula or chopper, or surgical instrument.

### SUMMARY

The invention is defined in the appended claims.

There is provided in accordance with an embodiment of the present disclosure, a phacoemulsification system, including a phacoemulsification probe including a distal end including a needle, an irrigation channel configured to convey irrigation fluid to the distal end, an aspiration channel configured to convey eye fluid and waste matter away from the distal end, wherein the aspiration channel includes a first section and a second section, a valve including an inlet port and an outlet port, wherein the first section of the aspiration channel is coupled with the inlet port and the distal end, wherein the second section of the aspiration channel is coupled with the outlet port, and wherein the valve is configured to selectively control fluid connectivity in the aspiration channel between the inlet port and the outlet port, and a bypass channel coupled with the irrigation channel and the second section of the aspiration channel and configured to allow a portion of the irrigation fluid in the irrigation channel to enter the second section of the aspiration channel.

Further in accordance with an embodiment of the present disclosure, the system includes an aspiration tubing line configured to be coupled with the second section, and a pumping sub-system configured to be coupled with the aspiration tubing line and pump the eye fluid and waste matter away from the distal end via the aspiration tubing line and the aspiration channel.

Still further in accordance with an embodiment of the present disclosure the bypass channel is configured to allow the portion of the irrigation fluid in the irrigation channel to enter the second section of the aspiration channel even when the valve is closed.

The bypass channel provides a permanent fluid connection between the irrigation channel and the second section of the aspiration channel.

Moreover, in accordance with an embodiment of the present disclosure, the system includes a sensor configured to provide a signal indicative of a fluid metric in the second section of the aspiration channel, and a controller configured to selectively control the fluid connectivity between the inlet port and the outlet port responsively to the fluid metric.

Further in accordance with an embodiment of the present disclosure the fluid metric is a pressure level.

Still further in accordance with an embodiment of the present disclosure the controller is configured to detect a rate of change of the fluid metric in the second section of the aspiration channel, and reduce the fluid connectivity between the inlet port and the outlet port responsively to the detected rate of change passing a given rate of change.

Additionally, in accordance with an embodiment of the present disclosure the controller is configured to reduce the fluid connectivity between the inlet port and the outlet port by repeatedly opening and closing the valve.

Moreover, in accordance with an embodiment of the present disclosure the controller is configured to reduce the fluid connectivity between the inlet port and the outlet port responsively to the detected rate of change passing a given rate of change while the portion of the irrigation fluid in the irrigation channel enters the second section of the aspiration channel via the bypass channel increasing the fluid metric in the second section of the aspiration channel.

Further in accordance with an embodiment of the present disclosure the controller is configured to increase the fluid connectivity between the inlet port and the outlet port responsively to the fluid metric in the second section of the aspiration channel passing a given value.

Still further in accordance with an embodiment of the present disclosure the phacoemulsification probe further includes a probe body and a fluid dynamics cartridge configured to be removably connected to the probe body, the fluid dynamics cartridge including the valve, the sensor, and the bypass channel.

Additionally, in accordance with an embodiment of the present disclosure the fluid dynamics cartridge includes the controller.

There is also provided in accordance with still another embodiment of the present disclosure, a phacoemulsification fluid dynamics cartridge apparatus according to claim 12. A further embodiment is provided in claim 13.

### BRIEF DESCRIPTION OF THE DRAWINGS

The present invention will be understood from the following detailed description, taken in conjunction with the drawings in which:
Fig. 1 is a partly pictorial, partly block diagram view of a phacoemulsification system constructed and operative in accordance with an embodiment of the present invention;
Figs. 2A-B are views of a probe for use with the system of Fig. 1;
Fig. 3A is a schematic view of an interior of a fluid dynamics cartridge for use in the probe of Figs. 2A-B;
Fig. 3B is a cross-section of the fluid dynamics cartridge through line B:B of Fig. 3A;
Fig. 3C is a cross-section of the fluid dynamics cartridge through line C:C of Fig. 3A;
Figs. 4A-B are schematic views of a permanent magnet in a solenoid coil;
Figs. 5A-B are schematic views of operation of a solenoid valve for use in the cartridge of Figs. 3A-C; and
Fig. 6 is a flowchart including steps in a method of operation of system of Fig. 1.

### DESCRIPTION OF EXAMPLE EMBODIMENTS

### OVERVIEW

During phacoemulsification of an eye lens, the emulsified lens particles are aspirated. When a particle blocks the inlet of an aspiration channel (which could be in a needle of a phacoemulsification probe) causing occlusion of the channel, the vacuum in the channel increases. When the channel becomes unblocked (e.g., by the particle being subsequently sucked down the channel), the high vacuum in the channel causes an aspiration surge known as post occlusion surge, which may have traumatic consequences to the eye. For example, sensitive parts of the eye may be damaged or come into contact with the needle of the phacoemulsification probe.

A possible solution to the problem of vacuum level surge is incorporating an aspiration bypass. Such a bypass may consist of a small hole or channel between an irrigation channel of the probe and the aspiration channel. When a blockage occurs, the high vacuum diverts irrigation fluid into the aspiration channel via the hole, thereby limiting the vacuum level.

However, the above-described bypass aspiration technique is still prone to produce a traumatic aspiration surge when the channel unblocks, since the high vacuum is present in a long tube (which being flexible may also be compressed adding to the vacuum problem) between a portion of the aspiration channel inside the phacoemulsification probe and the aspiration pump, and that large, partially vacant volume, may therefore cause a surge when the occlusion breaks. Moreover, diversion of irrigation fluid may cause an uncontrolled pressure-drop in the irrigation channel, which may also pose a risk to the eye.

Embodiments of the present invention generally solve the above problems by providing a phacoemulsification probe including a valve to selectively control fluid connectivity in an aspiration channel of the probe, and a bypass channel connecting an irrigation channel of the probe to the aspiration channel. The aspiration channel includes two sections, a first section extending from a distal end of the probe to the valve, and a second section extending from the valve for connection to an aspiration tubing line, which is connected to an aspiration pump for aspirating eye fluid and waste matter away from the distal end of the probe via the aspiration channel and aspiration tubing line. The bypass channel is connected to the second section of the aspiration channel and may be described as being more proximal in the probe than the valve (depending on the tubing configuration in the probe).

In operation, upon detection of an occlusion or occlusion clearance, the valve is actuated quickly (e.g., within 10 milliseconds) to reduce fluid connectivity in the aspiration channel (e.g., by closing the valve), while in a non-time critical manner, irrigation fluid enters the second section of the aspiration channel via the bypass channel to reduce the vacuum in the second section of the aspiration channel and the aspiration tubing line. Once the pressure in the second section of the aspiration channel has passed (e.g., increased) above a given limit (e.g., the vacuum has reduced), the valve may then be reopened allowing fluid to be aspirated without causing a surge.

In some embodiments, the valve is implemented as a solenoid valve includes a solenoid coil which moves a plunger including a permanent magnet in a valve cavity. Two parts of the aspiration channel are connected to the valve cavity via ports in the valve cavity. Therefore, movement of the plunger in the valve cavity controls the fluid connectivity in the aspiration channel. In other embodiments, the valve may be any suitable non-solenoid valve.

The solenoid valve does not need a restoring element (such as a spring) to keep the plunger in a rest position when a current is not applied to the solenoid coil. An electric current needs to be applied to the solenoid coil to selectively open the valve and keep the valve open, and to close the valve and keep the valve closed. If a current is not supplied to the solenoid coil, the position of the plunger may be unstable and unknown. Using a solenoid valve without a restoring element allows the plunger to be moved quickly with a selected force, while minimizing electrical power needed to open or close the valve thereby reducing heat generated by the solenoid valve. The solenoid valve is opened and closed by changing the polarity of the solenoid coil by changing the direction of the current applied to the solenoid coil.

In some embodiments, a spacer is placed in the path of the plunger preventing a center of the permanent magnet of the plunger (with respect to a direction of elongation of the valve cavity) from being aligned with a center of the solenoid coil (with respect to a direction of elongation of the valve cavity). In this asymmetrical state, the permanent magnet is not subjected to unstable forces from the solenoid coil and the plunger can be moved from one position to another by changing the polarity of the solenoid coil thereby providing a quick and effective opening and closing of the solenoid valve.

In some embodiments, a sensor (e.g., a flow sensor or pressure sensor, or any suitable sensor) connected to or coupled with the aspiration channel provides a signal indicative of a fluid metric (e.g., pressure level) in the second section of the aspiration channel and a controller selectively controls fluid connectivity along the aspiration channel by applying a suitable current to the solenoid coil to selectively open or close the solenoid valve. In some embodiments, when the controller detects a rate of change in the fluid metric (e.g., pressure level) in the second section of the aspiration channel passing (e.g., exceeding) a given rate of change, which is indicative of an occlusion breaking, the controller reduces fluid connectivity in the aspiration channel by closing the solenoid valve quickly (for example, in 10 milliseconds or less) thereby isolating the eye from the vacuum in the second section of the aspiration channel and aspiration tubing line until the pressure in the aspiration channel and/or aspiration line returns to a desired and/or safe pressure. The pressure in the second section of the aspiration channel may be changed (e.g., increased to reduce the vacuum), in a non-time critical manner, via irrigation fluid from the irrigation channel entering the aspiration channel via the bypass channel as mentioned previously. Once the pressure in the second section of the aspiration channel passes (e.g., exceeds) a given value (e.g., given pressure level), the controller reopens the solenoid valve without causing a vacuum surge, which could damage the eye.

In some embodiments, in addition to being linear, the solenoid valve is small and may be produced at low-cost thereby allowing the valve to be disposed of after use. Therefore, in some embodiments, the valve does not need to withstand repeated sterilization. The valve may be housed in a cartridge which is removably connected to the phacoemulsification probe and aspiration and irrigation tubes. The cartridge may then be removed from the probe and tubes after use for cleaning or disposal.

In some embodiments, sensors (e.g., a pressure sensor for the aspiration channel and a pressure sensor for the irrigation channel) may be included in the cartridge). Including the sensors in the cartridge may provide higher sensitivity to local changes in fluid dynamics and provide a higher degree of control of the pressure in the eye.

In some embodiments, the controller is also included in the cartridge. Including the controller in the cartridge may allow the controller to be configured for the calibration of the solenoid valve. Additionally, or alternatively, including the controller in the cartridge allows the controller to be close to the sensor or sensors which may be providing analog signals that could degrade if the signals needed to travel over a cable to a remote console in which the controller may otherwise be installed.

### SYSTEM DESCRIPTION

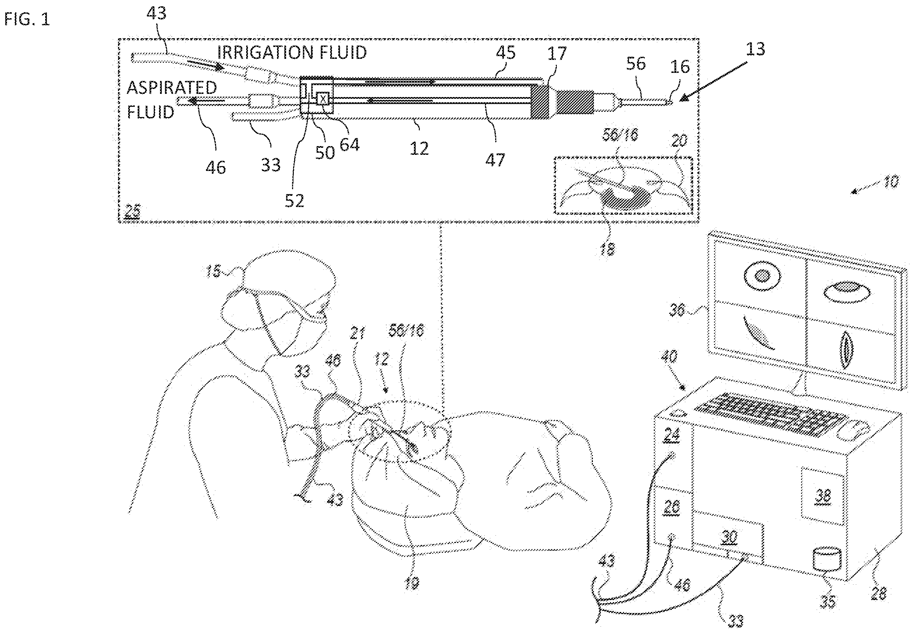

Reference is now made to Fig. 1 is a partly pictorial, partly block diagram view of a phacoemulsification system 10 constructed and operative in accordance with an embodiment of the present invention.

The phacoemulsification system 10 comprises a phacoemulsification probe 12 (e.g., handpiece). In some embodiments, the phacoemulsification probe 12 may be replaced by any suitable medical tool. As seen in the pictorial view of phacoemulsification system 10, and in inset 25, phacoemulsification probe 12 comprises: a distal end 13 including a needle 16; a probe body 17; and a coaxial irrigation sleeve 56 that at least partially surrounds needle 16 and creates a fluid pathway between the external wall of the needle and the internal wall of the irrigation sleeve, where needle 16 is hollow to provide an aspiration channel. Moreover, irrigation sleeve 56 may have one or more side ports at, or near, the distal end to allow irrigation fluid to flow towards the distal end of the phacoemulsification probe 12 through the fluid pathway and out of the port(s).

Needle 16 is configured for insertion into a lens capsule 18 of an eye 20 of a patient 19 by a physician 15 to remove a cataract. While the needle 16 (and irrigation sleeve 56) are shown in inset 25 as a straight object, any suitable needle may be used with phacoemulsification probe 12, for example, a curved or bent tip needle commercially available from Johnson & Johnson Surgical Vision, Inc., Santa Ana, CA, USA.

In the embodiment of Fig. 1, during the phacoemulsification procedure, a pumping sub-system 24 comprised in a console 28 pumps irrigation fluid from an irrigation reservoir (not shown) to the irrigation sleeve 56 to irrigate the eye 20. The irrigation fluid is pumped via an irrigation tubing line 43 running from the console 28 to an irrigation channel 45 of probe 12, the distal end of the irrigation channel 45 including the fluid pathway in the irrigation sleeve 56. The irrigation channel 45 is configured to convey the irrigation fluid to the distal end 13. The irrigation tubing line 43 is typically flexible and may be prone to collapsing during an occlusion of the needle 16. In another embodiment, the pumping sub-system 24 may be coupled or replaced with a gravity fed irrigation source such as a balanced salt solution (BSS) bottle/bag.

The phacoemulsification probe 12 includes an aspiration channel 47 configured to convey eye fluid and waste matter (e.g., emulsified parts of the cataract) away from the distal end 13. The aspiration channel 47 extends from the hollow of needle 16 through the phacoemulsification probe 12, and then via an aspiration tubing line 46 to a collection receptacle in the console 28. The phacoemulsification system 10 includes a pumping sub-system 26 disposed in the console 28 and configured to be coupled with the aspiration tubing line 46 and pump the eye fluid and waste matter away from the distal end 13 via the aspiration channel 47 and aspiration tubing line 46.

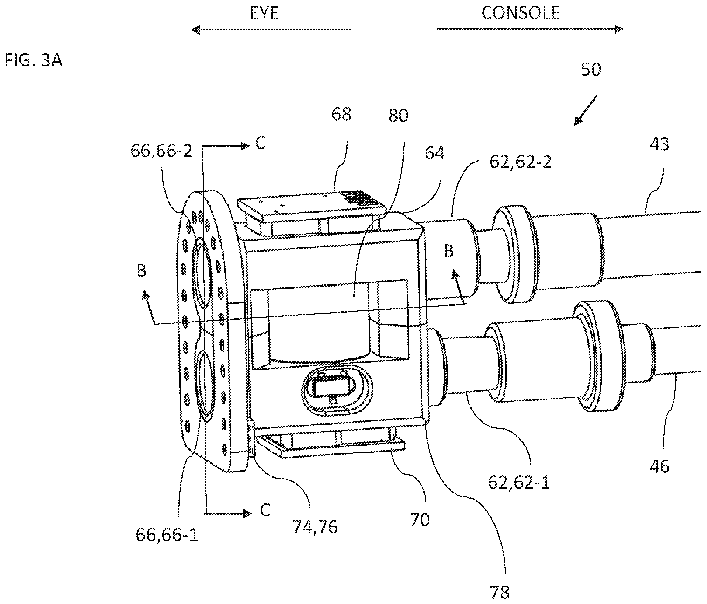

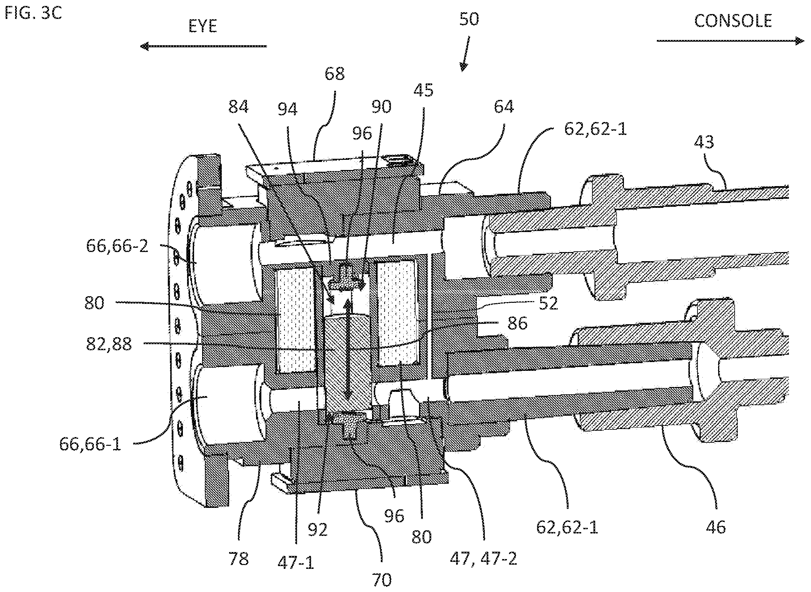

The phacoemulsification probe 12 includes a valve 64. The valve 64 is described herein as a solenoid valve. However, any suitable valve may replace the solenoid valve described herein. The valve 64 is described in more detail with reference to Figs. 3A-6. The phacoemulsification probe 12 includes a bypass channel 52 connected to the irrigation channel 45 and the aspiration channel 47, as described in more detail with reference to Fig. 3C.

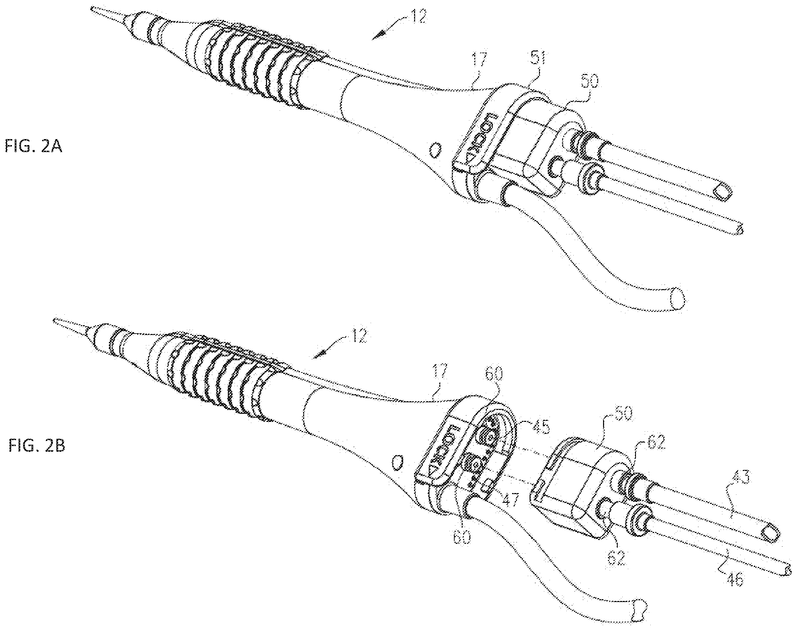

In some embodiments, the system 10 includes a fluid dynamics cartridge 50 (which may be configured to be removably coupled with the probe body 17 of the phacoemulsification probe 12), which includes the valve 64, the bypass channel 52, and sensors, described in more detail with reference to Figs. 2A-6. Part of the irrigation channel 45 and the aspiration channel 47 may be disposed in the probe body 17 and part disposed in the cartridge 50.

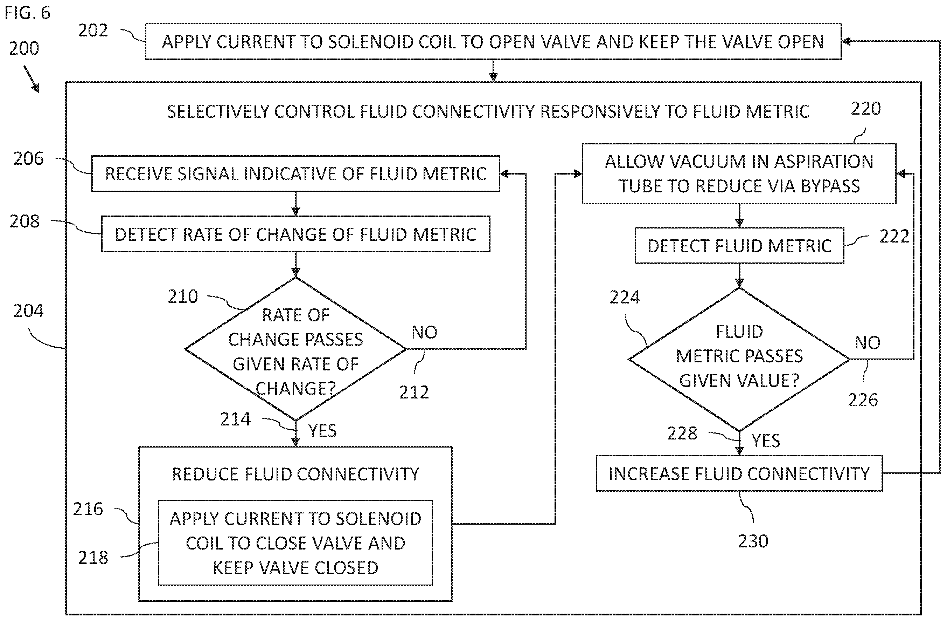

Phacoemulsification probe 12 includes other elements, such as a piezoelectric crystal (not shown) coupled with a horn (not shown) to drive vibration of needle 16. The piezoelectric crystal is configured to vibrate needle 16 in a resonant vibration mode. The vibration of needle 16 is used to break a cataract into small pieces during a phacoemulsification procedure. Console 28 comprises a piezoelectric drive module 30, coupled with the piezoelectric crystal, using electrical wiring running in a cable 33. Drive module 30 is controlled by a controller 38 and conveys processor-controlled driving signals via cable 33 to, for example, maintain needle 16 at maximal vibration amplitude. The drive module may be realized in hardware or software, for example, in a proportional-integral-derivative (PID) control architecture. The controller 38 may also be configured to receive signals from sensors in the phacoemulsification probe 12 and control one or more valves to regulate the flow of fluid in the irrigation channel 45 and/or the aspiration channel 47, as described in more detail with reference to Fig. 6. In some embodiments, at least some of the functionality of the controller 38 may be implemented using a controller disposed in the phacoemulsification probe 12 (e.g., the cartridge 50).

Controller 38 may receive user-based commands via a user interface 40, which may include setting a vibration mode and/or frequency of the piezoelectric crystal (not shown), and setting or adjusting an irrigation and/or aspiration rate of the pumping sub-systems 24, 26. In some embodiments, user interface 40 and a display 36 may be combined as a single touch screen graphical user interface. In some embodiments, the physician 15 uses a foot pedal (not shown) as a means of control. Additionally, or alternatively, controller 38 may receive the user-based commands from controls located in a handle 21 of probe 12.

Some or all of the functions of controller 38 may be combined in a single physical component or, alternatively, implemented using multiple physical components. These physical components may comprise hard-wired or programmable devices, or a combination of the two. In some embodiments, at least some of the functions of controller 38 may be carried out by suitable software stored in a memory 35 (as shown in Fig. 1). This software may be downloaded to a device in electronic form, over a network, for example. Alternatively, or additionally, the software may be stored in tangible, non-transitory computer-readable storage media, such as optical, magnetic, or electronic memory.

The system shown in Fig. 1 may include further elements which are omitted for clarity of presentation. For example, physician 15 typically performs the procedure using a stereo-microscope or magnifying glasses, neither of which are shown. Physician 15 may use other surgical tools in addition to probe 12, which are also not shown in order to maintain clarity and simplicity of presentation.

Reference is now made to Figs. 2A-B, which are views of the phacoemulsification probe 12 for use with the system 10 of Fig. 1. Fig. 2A shows the cartridge 50, which is configured to be removably coupled (using a clip 51) with the probe body 17 of the phacoemulsification probe 12. Fig. 2B shows the cartridge 50 detached from the probe body 17. Fig. 2B shows ports 60 of the irrigation channel 45 and the aspiration channel 47 on the probe body 17 for connecting to corresponding ports (not shown in Fig. 2B, but shown in Fig. 3A) of the cartridge 50. Fig. 2B also shows irrigation tubing line 43 and aspiration tubing line 46 connected to ports 62 of the cartridge 50.

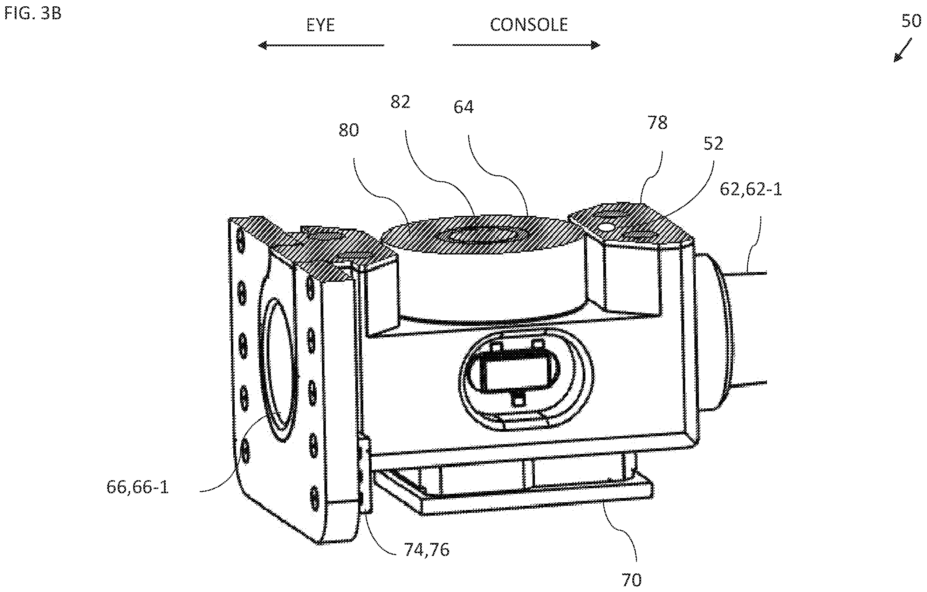

Reference is now made to Figs. 3A-C. Fig. 3A is a schematic view of an interior of a fluid dynamics cartridge 50 for use in the phacoemulsification probe 12 of Figs. 2A-B. Fig. 3B is a cross-section of the fluid dynamics cartridge 50 through line B:B of Fig. 3A. Fig. 3C is a cross-section of the fluid dynamics cartridge 50 through line C:C of Fig. 3A.

The phacoemulsification probe 12 may include sensors 68, and 70 (which may be pressure sensors or flow sensors or any suitable sensors), and a solenoid valve 64. In some embodiments, the cartridge 50 includes: the solenoid valve 64, ports 62 for connection to the irrigation tubing line 43 and aspiration tubing line 46, ports 66 for connection to the ports 60 (Fig. 2B), and sections of the irrigation channel 45 and aspiration channel 47; the bypass channel 52 on the console 28 side of the solenoid valve 64; the sensor 68 connected to the irrigation channel 45; and the sensor 70 connected to aspiration channel 47 on the console 28 side of the solenoid valve 64 (as shown in Fig. 3C). The sensor 68 and the sensor 70 are configured to provide respective signals indicative of respective fluid metrics (e.g., pressure levels) in the irrigation channel 45 and in a section 47-2 (Fig. 3C) of the aspiration channel 47. The aspiration channel 47 traverses the solenoid valve 64.

Including the sensors 68, 70 in the cartridge 50 may provide higher sensitivity to local changes in fluid dynamics and provide a higher degree of control of the pressure in the eye.

The phacoemulsification probe 12 may include a controller 74 to receive the signal(s) from the pressure sensor 68 and/or the pressure sensor 70, and control the fluid connectivity in the irrigation channel 45 and/or the aspiration channel 47 by selectively opening and closing the solenoid valve 64, responsively to the received signal(s). In some embodiments, the cartridge 50 may also include the controller 74 and/or a memory 76 (e.g., EEPROM) to hold calibration settings and/or a usage counter to count usage of the cartridge 50 and thereby prevent overuse of the cartridge 50. In some embodiments, the controller 74 may be included in the console 28 (Fig. 1). In some embodiments, the functionality of the controller 74 may be performed by the controller 38. Including the controller 74 in the cartridge 50 may allow the controller to be configured for the calibration of the solenoid valve 64. Additionally, or alternatively, including the controller 74 in the cartridge 50 allows the controller to be close to the sensors 68, 70 which may be providing analog signals that could degrade if the signals needed to travel over the cable 33 to the console 28 in which the controller 74 may otherwise be installed.

The cartridge 50 is compact and may be any suitable size. In some embodiments, the cartridge 50 may fit into a cube of 2.5 cm sides.

In some embodiments, the cartridge 50 includes an aspiration inlet port 66-1 and an aspiration outlet port 62-1. The aspiration inlet port 66-1 is configured to be removably coupled with the aspiration channel 47 of the probe body 17 of the phacoemulsification probe 12 via one of the ports 60. The aspiration outlet port 62-1 is configured to be coupled with the aspiration tubing line 46. The removable cartridge 50 also includes a section of the aspiration channel 47 fluidically connecting the aspiration inlet port 66-1 to the aspiration outlet port 62-1. The removable cartridge 50 also includes an irrigation inlet port 62-2 and an irrigation outlet port 66-2. The irrigation inlet port 62-2 is configured to be coupled with the irrigation tubing line 43. The irrigation outlet port 66-2 is configured to be removably coupled with the irrigation channel 45 of the probe body 17 of the phacoemulsification probe 12 via one of the ports 60. The removable cartridge 50 also includes a section of the irrigation channel 45 fluidically connecting the irrigation inlet port 62-2 to the irrigation outlet port 66-2.

The removable cartridge 50 also includes the solenoid valve 64 disposed in the section of the aspiration channel 47 between the aspiration inlet port 66-1 and the aspiration outlet port 62-1. The aspiration channel 47 may be defined to include: a section 47-1 coupled with the aspiration inlet port 66-1 and the distal end 13 (Fig. 1 and 3C); and section 47-2 coupled with the aspiration outlet port 62-1 (as shown in Fig. 3C). The solenoid valve 64 is configured to selectively control fluid connectivity in the aspiration channel 47 between the aspiration inlet port 66-1 and the aspiration outlet port 62-1. The section 47-2 is configured to be coupled with the aspiration tubing line 46 via the outlet port 62-1.

The controller 74 is configured to selectively control the fluid connectivity between the aspiration inlet port 66-1 and the aspiration outlet port 62-1 by selectively opening and closing the solenoid valve 64, responsively to a fluid metric (e.g., a sensed fluid flow or pressure level) in section 47-2 of the aspiration channel 47. It should be noted that when the solenoid valve 64 is closed, the sensor 70 shown in Fig. 3C is configured to sense the fluid metric (e.g., a fluid flow or pressure level) in the section 47-2 between the solenoid valve 64 and the console 28.

The cartridge 50 also includes the bypass channel 52 fluidically connecting the section of the irrigation channel 45 in the cartridge 50 to the section 47-2 of the aspiration channel 47 (at a region between the solenoid valve 64 and the aspiration outlet port 62-1). The bypass channel 52 is configured to allow a portion of the irrigation fluid in the irrigation channel 45 to enter the section 47-2 of the aspiration channel 47 even when the solenoid valve 64 is closed, as described in more detail below with reference to Fig. 6. In disclosed embodiments, the bypass channel 52 does not include a valve and provides a permanent fluid connection between the irrigation channel 45 and the section 47-2 of the aspiration channel 47.

The solenoid valve 64 and its operation is now described in more detail. The solenoid valve 64 includes a valve body 78, a solenoid coil 80, and a plunger 82.

Reference is now made to Fig. 3C. The valve body 78 includes the ports 62, the ports 66, a valve cavity 84 having a direction of elongation 86 and configured to provide fluid connectivity between respective ones of the ports 62, 66 (e.g., between the aspiration inlet port 66-1 and aspiration outlet port 62-1). The solenoid coil 80 is disposed in the valve body 78 around valve cavity 84. The plunger 82 includes a permanent magnet 88. The permanent magnet 88 may comprise all of, or only part of, the plunger 82. For example, the plunger 82 may include the permanent magnet 88 coated or covered with a material of low friction. The plunger 82 is configured to move back-and-forth along the direction of elongation 86 between a position 90 and a position 92 in the valve cavity 84 selectively controlling the fluid connectivity between respective ones of the ports 62, 66 (e.g., between the aspiration inlet port 66-1 and aspiration outlet port 62-1).

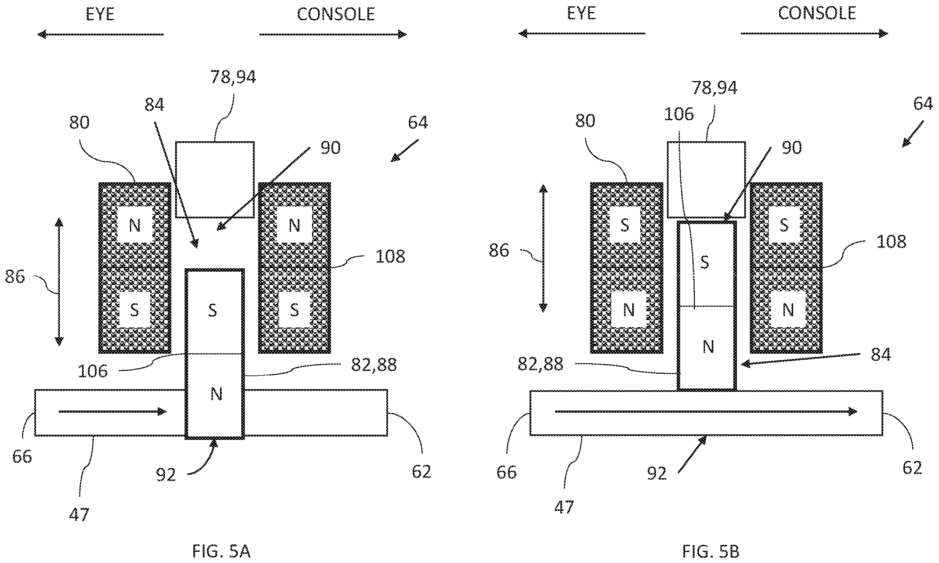

The plunger 82 may have any suitable size, for example, a length in the range of 3mm to 2 cm (e.g., 6 mm) and a diameter in the range of 1mm to 1cm (e.g., 3 mm). The valve body 78 may include a spacer 94 described in more detail with reference to Figs 5A-B below. The valve body 78 may also include one or more shock absorbers 96 to soften banging of the plunger 82 against the valve body 78. In Fig. 3C, the upper shock absorber 96 forms part of the spacer 94.

The controller 74 (Figs. 3A-B) is configured to apply at least one current to the solenoid coil 80 to selectively move the plunger 82 between the position 90 and the position 92, and to selectively maintain the plunger in the position 90 and the position 92, as described below in more detail with reference to Figs. 5A-B.

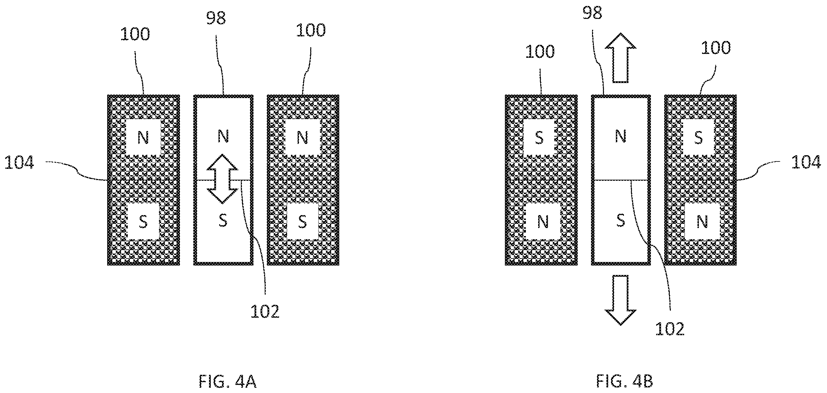

Reference is now made to Figs. 4A-B, which are schematic views of a permanent magnet 98 in a solenoid coil 100.

In the configuration of Fig. 4A, the polarity of the solenoid coil 100 is in the same direction as the polarity of the permanent magnet 98. In such a configuration, if a center 102 of the permanent magnet 98 is moved a little away from a center 104 of the solenoid coil 100, the permanent magnet 98 will oscillate around the center 104 of the solenoid coil 100 until the permanent magnet 98 settles so that the center 102 of the permanent magnet 98 is aligned with the center 104 of the solenoid coil 100. The permanent magnet 98 therefore rests in a stable position with respect to the solenoid coil 100.

In the configuration of Fig. 4B, the polarity of the solenoid coil 100 is in the opposite direction to the polarity of the permanent magnet 98. In such a configuration, if the center 102 of the permanent magnet 98 is moved a little away from the center 104 of the solenoid coil 100, the permanent magnet 98 will continue to move in that direction. The permanent magnet 98 in Fig. 4B is therefore in an unstable position with respect to the solenoid coil 100.

Reference is now made to Figs. 5A-B, which are schematic views of operation of the solenoid valve 64 for use in the cartridge 50 of Figs. 3A-C.

The plunger 82 is configured to move back-and-forth along the direction of elongation 86 between position 92 and position 90 in the valve cavity 84 selectively controlling the fluid connectivity between respective ones of the ports 66, 62. The controller 74 (Figs. 3A-B) is configured to apply current to the solenoid coil 80 to selectively move the plunger 82 between the position 92 and position 90, and to selectively maintain the plunger in the position 92 and position 90. Fig. 5A shows the plunger 82 in position 92 blocking fluid connectivity in the aspiration channel 47. Fig. 5B shows the plunger 82 in position 90 allowing fluid connectivity in the aspiration channel 47.

The plunger 82 does not have a fixed rest position in the valve cavity 84. Even though in some orientations the plunger 82 may fall in one of the positions 92, 94 due to gravity, if the solenoid valve 64 is orientated differently the plunger 84 may fall to a different position. The plunger 82 does not include a restoring element (e.g., spring) configured to restore the plunger 82 to a fixed rest position. The plunger will not always remain in the position 92 or position 90 (e.g., if the orientation of the phacoemulsification probe 12 is changed) without applying current to the solenoid coil 80. In other words, for the solenoid valve 64 to function correctly, a current is applied to the solenoid coil 80 whether the solenoid valve 64 is to remain open or closed. The plunger 82 will remain in the position 90 or the position 92 upon application of current to the solenoid coil 80.

The controller 74 is configured to apply a current to the solenoid coil 80 to activate the solenoid coil 80 with a polarity to cause the plunger 82 to move and be maintained in the position 92 as shown in Fig. 5A. The controller 74 is configured to apply an opposite current to the solenoid coil 80 to activate the solenoid coil 80 with an opposite polarity to cause the plunger 82 to move and be maintained in the position 90 as shown in Fig. 5B.

The permanent magnet 88 has a center 106 with respect to the direction of elongation 86. The solenoid coil 80 has a center 108 with respect to the direction of elongation 86.

The valve body 78 includes the spacer 94 to prevent the center 106 of the magnet 88 from moving in the direction of elongation 86 past the center 108 of the solenoid coil 80. Therefore, the spacer 94 maintains asymmetry between the center 108 of the solenoid coil 80 and the center 106 of the permanent magnet 88 with respect to the direction of elongation 86 so that the centers 106, 108 are never aligned with respect to the direction of elongation 86. The above asymmetry is desirable to allow movement of the permanent magnet 88 within the valve cavity 84 to be controlled and the maintained position of the permanent magnet 88 at position 90 to be stable (as explained above with reference to Figs. 4A-B) When plunger 82 is in position 90, plunger 82 abuts spacer 94 (see Fig. 5B).

Reference is now made to Fig. 6, which is a flowchart 200 including steps in an exemplary method of operation of system 10 of Fig. 1. Reference is also made to Fig. 3C.

The controller 74 is configured to apply (block 202) a current to the solenoid coil 80 to activate the solenoid coil 80 with a polarity to cause the plunger 82 to move and be maintained in the position 90 so that the solenoid valve 64 is open (and kept open) and there is fluid connectivity along the aspiration channel 47.

The controller 74 is configured to selectively control (block 204) the fluid connectivity responsively to a fluid metric (e.g., a sensed fluid flow or pressure level) from the one or more sensors 68, 70 coupled with aspiration channel 47. In this embodiment, the sensor(s) detect a change in pressure, but this method is applicable to other types of sensors known in the art. The step of block 204 is now described in more detail with reference to sub-steps of blocks 206-230.

The controller 74 is configured to receive a signal indicative of the fluid metric (e.g., pressure level) in the section 47-2 of the aspiration channel 47 from the sensor 70 (block 206). The controller 74 is configured to detect a rate of change of the fluid metric (e.g., pressure level) in the section 47-2 of the aspiration channel 47 responsively to the received signal (block 208). At a decision block 210, the controller 74 is configured to determine if the rate of change passes (e.g., exceeds) a given rate of change. If the rate of change does not pass (e.g., exceed) the given rate of change (branch 212), the method returns to the sub-step of block 206. If the rate of change passes (e.g., exceeds) the given rate of change (branch 214), the controller 74 is configured to reduce the fluid connectivity (block 216) between the aspiration inlet port 66-1 and the aspiration outlet port 62-1. The sub-step of block 216 may include the controller 74 being configured to apply a current to the solenoid coil 80 to activate the solenoid coil 80 with an opposite polarity to cause the plunger 82 to move and be maintained in the position 92 (block 218). The solenoid valve 64 is closed and kept closed thereby blocking fluid connectivity in the aspiration channel 47 at the location of the plunger 82 thereby isolating the eye from the aspiration tubing line 46 (Fig. 1) and protecting the eye from a vacuum surge.

In some embodiments, rather than the solenoid valve 64 closing completely and fast, the solenoid valve 64 may be controlled to close partially and/or slowly. In some embodiments, the activation of the solenoid valve 64 may also be controlled according to pressure, flow, temperature, or a combination of these type of sensed parameters.

While the step of block 216 is being performed, the vacuum in the aspiration tubing line 46 and the section 47-2 of the aspiration channel 47 between the solenoid valve 64 and the aspiration tubing line 46 is allowed to reduce (block 220) via a portion of the irrigation fluid in the irrigation channel 45 entering the section 47-2 of the aspiration channel 47 via the bypass channel 52 increasing the fluid metric (e.g., pressure level) in the section 47-2 of the aspiration channel 47. The controller 74 is configured to detect the fluid metric (e.g., pressure level) in the section 47-2 of the aspiration channel 47 responsively to signal received from the sensor 70 (block 222). At a decision block 224, the controller 74 is configured to determine if the fluid metric (e.g., pressure level) passes (e.g., exceeds) a given value (e.g., given pressure level). If fluid metric (e.g., pressure level) does not pass (e.g., exceed) the given value (e.g., given pressure level) (branch 226), the sub-step of block 220 is repeated. If the fluid metric (e.g., pressure level) passes (e.g., exceeds) the given value (e.g., given pressure level) (branch 228), the controller 74 is configured to increase (block 230) the fluid connectivity between the aspiration inlet port 66-1 and the aspiration outlet port 62-1 responsively to the fluid metric (e.g., pressure level) in the section 47-2 of the aspiration channel 47 passing (e.g., exceeding) a given value (e.g., given pressure level), for example, the step of block 202 is repeated.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable subcombination.

The embodiments described above are cited by way of example, and the present invention is not limited by what has been particularly shown and described hereinabove. Rather the scope of the invention, which is defined by the appended claims, includes both combinations and sub-combinations of the various features described hereinabove, as well as variations and modifications thereof which would occur to persons skilled in the art upon reading the foregoing description and which are not disclosed in the prior art.

## Claims

1. A phacoemulsification system (10), comprising a phacoemulsification probe (12) including:
a distal end (13) comprising a needle (16);
an irrigation channel (45) configured to convey irrigation fluid to the distal end;
an aspiration channel (47) configured to convey eye fluid and waste matter away from the distal end, wherein the aspiration channel comprises a first section (47-1) and a second section (47-2);
an inlet port (66-1) and an outlet port (62-1), wherein the first section of the aspiration channel is coupled with the inlet port and the distal end, and wherein the second section of the aspiration channel is coupled with the outlet port;
a valve (64) configured to selectively control fluid connectivity in the aspiration channel between the inlet port and the outlet port; and
a bypass channel (52) coupled with the irrigation channel and the second section of the aspiration channel and configured to allow a portion of the irrigation fluid in the irrigation channel to enter the second section of the aspiration channel;
**characterised in that** the bypass channel provides a permanent fluid connection between the irrigation channel and the second section of the aspiration channel.

2. The system according to claim 1, further comprising:
an aspiration tubing line (46) configured to be coupled with the second section; and
a pumping sub-system (24) configured to be coupled with the aspiration tubing line and pump the eye fluid and waste matter away from the distal end via the aspiration tubing line and the aspiration channel.

3. The system according to claim 1, wherein the bypass channel is configured to allow the portion of the irrigation fluid in the irrigation channel to enter the second section of the aspiration channel even when the valve is closed.

4. The system according to claim 1, further comprising:
a sensor (70) configured to provide a signal indicative of a fluid metric in the second section of the aspiration channel; and
a controller (38) configured to selectively control the fluid connectivity between the inlet port and the outlet port responsively to the fluid metric.

5. The system according to claim 4, wherein the fluid metric is a pressure level.

6. The system according to claim 4, wherein the controller is configured to detect a rate of change of the fluid metric in the second section of the aspiration channel, and reduce the fluid connectivity between the inlet port and the outlet port responsively to the detected rate of change passing a given rate of change.

7. The system according to claim 6, wherein the controller is configured to reduce the fluid connectivity between the inlet port and the outlet port by repeatedly opening and closing the valve.

8. The system according to claim 6, wherein the controller is configured to reduce the fluid connectivity between the inlet port and the outlet port responsively to the detected rate of change passing a given rate of change while the portion of the irrigation fluid in the irrigation channel enters the second section of the aspiration channel via the bypass channel increasing the fluid metric in the second section of the aspiration channel.

9. The system according to claim 8, wherein the controller is configured to increase the fluid connectivity between the inlet port and the outlet port responsively to the fluid metric in the second section of the aspiration channel passing a given value.

10. The system according to claim 4, wherein the phacoemulsification probe further comprises a probe body (17) and a fluid dynamics cartridge (50) configured to be removably connected to the probe body, the fluid dynamics cartridge comprising the valve, the sensor, and the bypass channel.

11. The system according to claim 10, wherein the fluid dynamics cartridge comprises the controller.

12. A phacoemulsification fluid dynamics cartridge apparatus (50) configured to be removably coupled with a phacoemulsification probe (12), and comprising:
an aspiration inlet port (66-1) and an aspiration outlet port (62-1), the aspiration inlet port being configured to be removably coupled with an aspiration channel (47) of the phacoemulsification probe, the aspiration outlet port being configured to be coupled with an aspiration tubing line (46);
an aspiration channel section fluidically connecting the aspiration inlet port to the aspiration outlet port, wherein the aspiration channel section comprises a first section (47-1) coupled with the aspiration inlet port and configured to be coupled with a distal end (13) of the phacoemulsification probe, wherein the aspiration channel section comprises a second section (47-2) coupled with the aspiration outlet port;
an irrigation inlet port (62-2) and an irrigation outlet port (66-2), the irrigation inlet port being configured to be coupled with an irrigation tubing line (43), the irrigation outlet port being configured to be removably coupled with an irrigation channel (45) of the phacoemulsification probe;
an irrigation channel section fluidically connecting the irrigation inlet port to the irrigation outlet port;
a valve (64) disposed in the aspiration channel section and configured to selectively control fluid connectivity in the aspiration channel section between the aspiration inlet port and the aspiration outlet port; and
a bypass channel (52) configured to allow a portion of the irrigation fluid in the irrigation channel section to enter the aspiration channel section;
**characterized in that** the bypass channel provides a permanent fluid connection between the irrigation channel section and the second section of the aspiration channel section.

13. The apparatus according to claim 12, wherein the bypass channel fluidically connects the irrigation channel section to the aspiration channel section at a region (47-2) between the valve and the aspiration outlet port.

## Patentansprüche

1. Phakoemulsifikationssystem (10), umfassend eine Phakoemulsifikationssonde (12), die Folgendes einschließt:
ein distales Ende (13), das eine Nadel (16) umfasst;
einen Spülkanal (45), der konfiguriert ist, um Spülfluid zu dem distalen Ende zu leiten;
einen Absaugkanal (47), der konfiguriert ist, um Augenfluid und Abfallmaterial von dem distalen Ende weg zu transportieren, wobei der Absaugkanal einen ersten Abschnitt (47-1) und einen zweiten Abschnitt (47-2) umfasst;
einen Einlassanschluss (66-1) und einen Auslassanschluss (62-1), wobei der erste Abschnitt des Absaugkanals mit dem Einlassanschluss und dem distalen Ende gekoppelt ist, und wobei der zweite Abschnitt des Absaugkanals mit dem Auslassanschluss gekoppelt ist;
ein Ventil (64), das konfiguriert ist, um die Fluidverbindbarkeit in dem Absaugkanal zwischen dem Einlassanschluss und dem Auslassanschluss selektiv zu steuern; und
einen Bypass-Kanal (52), der mit dem Spülkanal und dem zweiten Abschnitt des Absaugkanals gekoppelt ist und konfiguriert ist, um einem Teil des Spülfluids in dem Spülkanal zu ermöglichen, in den zweiten Abschnitt des Absaugkanals einzutreten;
**dadurch gekennzeichnet, dass** der Bypass-Kanal eine permanente Fluidverbindung zwischen dem Spülkanal und dem zweiten Abschnitt des Absaugkanals bereitstellt.

2. System nach Anspruch 1, ferner umfassend:
eine Absaugschlauchleitung (46), die konfiguriert ist, um mit dem zweiten Abschnitt gekoppelt zu werden; und
ein Pumpen-Untersystem (24), das konfiguriert ist, um mit der Absaugschlauchleitung gekoppelt zu werden und die Augenfluid und Abfallmaterial von dem distalen Ende über die Absaugschlauchleitung und den Absaugkanal weg zu pumpen.

3. System nach Anspruch 1, wobei der Bypass-Kanal konfiguriert ist, um dem Teil des Spülfluids in dem Spülkanal zu ermöglichen, in den zweiten Abschnitt des Absaugkanals einzutreten, selbst wenn das Ventil geschlossen ist.

4. System nach Anspruch 1, ferner umfassend:
einen Sensor (70), der konfiguriert ist, um ein Signal bereitzustellen, das eine Fluidmetrik in dem zweiten Abschnitt des Absaugkanals anzeigt; und
eine Steuerung (38), die konfiguriert ist, um die Fluidverbindbarkeit zwischen dem Einlassanschluss und dem Auslassanschluss als Reaktion auf die Fluidmetrik selektiv zu steuern.

5. System nach Anspruch 4, wobei die Fluidmetrik ein Druckniveau ist.

6. System nach Anspruch 4, wobei die Steuerung konfiguriert ist, um eine Änderungsrate der Fluidmetrik in dem zweiten Abschnitt des Absaugkanals zu detektieren und die Fluidverbindbarkeit zwischen dem Einlassanschluss und dem Auslassanschluss als Reaktion darauf zu reduzieren, dass die detektierte Änderungsrate eine gegebene Änderungsrate überschreitet.

7. System nach Anspruch 6, wobei die Steuerung konfiguriert ist, um die Fluidverbindbarkeit zwischen dem Einlassanschluss und dem Auslassanschluss durch wiederholtes Öffnen und Schließen des Ventils zu reduzieren.

8. System nach Anspruch 6, wobei die Steuerung konfiguriert ist, um die Fluidverbindbarkeit zwischen dem Einlassanschluss und dem Auslassanschluss als Reaktion darauf zu reduzieren, dass die detektierte Änderungsrate eine gegebene Änderungsrate überschreitet, während der Teil des Spülfluids in dem Spülkanal über den Bypass-Kanal in den zweiten Abschnitt des Absaugkanals eintritt und dabei die Fluidmetrik in dem zweiten Abschnitt des Absaugkanals erhöht.

9. System nach Anspruch 8, wobei die Steuerung konfiguriert ist, um die Fluidverbindbarkeit zwischen dem Einlassanschluss und dem Auslassanschluss als Reaktion darauf zu erhöhen, dass die Fluidmetrik in dem zweiten Abschnitt des Absaugkanals einen gegebenen Wert überschreitet.

10. System nach Anspruch 4, wobei die Phakoemulsifikationssonde ferner einen Sondenkörper (17) und eine Fluiddynamikkartusche (50) umfasst, die konfiguriert ist, um lösbar mit dem Sondenkörper verbunden zu werden, wobei die Fluiddynamikkartusche das Ventil, den Sensor und den Bypass-Kanal umfasst.

11. System nach Anspruch 10, wobei die Fluiddynamikkartusche die Steuerung umfasst.

12. Phakoemulsifikationsfluidynamikkartuscheneinrichtung (50), die konfiguriert ist, um lösbar mit einer Phakoemulsifikationssonde (12) gekoppelt zu werden, und umfassend:
einen Absaugeinlassanschluss (66-1) und einen Absaugauslassanschluss (62-1), wobei der Absaugeinlassanschluss konfiguriert ist, um lösbar mit einem Absaugkanal (47) der Phakoemulsifikationssonde gekoppelt zu werden, wobei die Absaugauslassanschluss konfiguriert ist, um mit einer Absaugschlauchleitung (46) gekoppelt zu werden;
ein Absaugkanalsegment, das den Absaugeinlassanschluss fluidisch mit dem Absaugauslassanschluss verbindet, wobei das Absaugkanalsegment einen ersten Abschnitt (47-1) umfasst, der mit dem Absaugeinlassanschluss gekoppelt und konfiguriert ist, um mit einem distalen Ende (13) der Phakoemulsifikationssonde gekoppelt zu werden, wobei das Absaugkanalsegment einen zweiten Abschnitt (47-2) umfasst, der mit dem Absaugauslassanschluss gekoppelt ist;
einen Spüleinlassanschluss (62-2) und einen Spülauslassanschluss (66-2), wobei der Spüleinlassanschluss konfiguriert ist, um mit einer Spülschlauchleitung (43) gekoppelt zu werden, wobei der Spülauslassanschluss konfiguriert ist, um lösbar mit einem Spülkanal (45) der Phakoemulsifikationssonde gekoppelt zu werden;
einen Spülkanalabschnitt, der den Spüleinlassanschluss fluidisch mit dem Spülauslassanschluss verbindet;
ein Ventil (64), das in dem Absaugkanalabschnitt angeordnet und konfiguriert ist, um die Fluidverbindbarkeit in dem Absaugkanalabschnitt zwischen dem Absaugeinlassanschluss und dem Absaugauslassanschluss selektiv zu steuern; und
einen Bypass-Kanal (52), der konfiguriert ist, um einem Teil des Spülfluids in dem Spülkanalabschnitt zu ermöglichen, in den Absaugkanalabschnitt einzutreten;
**dadurch gekennzeichnet, dass** der Bypass-Kanal eine permanente Fluidverbindung zwischen dem Spülkanalabschnitt und dem zweiten Abschnitt des Absaugkanalabschnitts bereitstellt.

13. Einrichtung nach Anspruch 12, wobei der Bypass-Kanal den Spülkanalabschnitt mit dem Absaugkanalabschnitt in einem Bereich (47-2) zwischen dem Ventil und dem Absaugauslassanschluss fluidisch verbindet.

## Revendications

1. Système de phacoémulsification (10), comprenant une sonde de phacoémulsification (12) comportant :
une extrémité distale (13) comprenant une aiguille (16) ;
un canal d'irrigation (45) conçu pour transporter un fluide d'irrigation vers l'extrémité distale ;
un canal d'aspiration (47) conçu pour transporter un fluide oculaire et des matières résiduelles à l'écart de l'extrémité distale, dans lequel le canal d'aspiration comprend une première section (47-1) et une seconde section (47-2) ;
un orifice d'entrée (66-1) et un orifice de sortie (62-1), dans lequel la première section du canal d'aspiration est accouplée à l'orifice d'entrée et à l'extrémité distale, et dans lequel la seconde section du canal d'aspiration est accouplée à l'orifice de sortie ;
une valve (64) conçue pour commander sélectivement une connectivité de fluide dans le canal d'aspiration entre l'orifice d'entrée et l'orifice de sortie ; et
un canal de dérivation (52) accouplé au canal d'irrigation et à la seconde section du canal d'aspiration et conçu pour permettre à une partie du fluide d'irrigation dans le canal d'irrigation d'entrer dans la seconde section du canal d'aspiration ;
**caractérisé en ce que** le canal de dérivation fournit un raccordement fluidique permanent entre le canal d'irrigation et la seconde section du canal d'aspiration.

2. Système selon la revendication 1, comprenant en outre :
une conduite tubulaire d'aspiration (46) conçue pour être accouplée à la seconde section ; et
un sous-système de pompage (24) conçu pour être accouplé à la conduite tubulaire d'aspiration et pour pomper le fluide oculaire et les matières résiduelles à l'écart de l'extrémité distale par l'intermédiaire de la conduite tubulaire d'aspiration et du canal d'aspiration.

3. Système selon la revendication 1, dans lequel le canal de dérivation est conçu pour permettre à la partie du fluide d'irrigation dans le canal d'irrigation d'entrer dans la seconde section du canal d'aspiration même lorsque la valve est fermée.

4. Système selon la revendication 1, comprenant en outre :
un capteur (70) configuré pour fournir un signal indiquant une métrique de fluide dans la seconde section du canal d'aspiration ; et
un organe de commande (38) configuré pour commander sélectivement la connectivité de fluide entre l'orifice d'entrée et l'orifice de sortie en réponse à la métrique de fluide.

5. Système selon la revendication 4, dans lequel la métrique de fluide est un niveau de pression.

6. Système selon la revendication 4, dans lequel l'organe de commande est configuré pour détecter un taux de changement de la métrique de fluide dans la seconde section du canal d'aspiration, et pour réduire la connectivité de fluide entre l'orifice d'entrée et l'orifice de sortie en réponse au taux de changement détecté dépassant un taux de changement donné.

7. Système selon la revendication 6, dans lequel l'organe de commande est configuré pour réduire la connectivité de fluide entre l'orifice d'entrée et l'orifice de sortie en ouvrant et en fermant de manière répétée la valve.

8. Système selon la revendication 6, dans lequel l'organe de commande est configuré pour réduire la connectivité de fluide entre l'orifice d'entrée et l'orifice de sortie en réponse au taux de changement détecté dépassant un taux de changement donné alors que la partie du fluide d'irrigation dans le canal d'irrigation entre dans la seconde section du canal d'aspiration par l'intermédiaire du canal de dérivation augmentant la métrique de fluide dans la seconde section du canal d'aspiration.

9. Système selon la revendication 8, dans lequel l'organe de commande est configuré pour augmenter la connectivité de fluide entre l'orifice d'entrée et l'orifice de sortie en réponse à la métrique de fluide dans la seconde section du canal d'aspiration dépassant une valeur donnée.

10. Système selon la revendication 4, dans lequel la sonde de phacoémulsification comprend en outre un corps de sonde (17) et une cartouche de dynamique de fluide (50) conçue pour être raccordée de manière amovible au corps de sonde, la cartouche de dynamique de fluide comprenant la valve, le capteur et le canal de dérivation.

11. Système selon la revendication 10, dans lequel la cartouche de dynamique de fluide comprend l'organe de commande.

12. Appareil de cartouche de dynamique de fluide (50) de phacoémulsification conçu pour être accouplé de manière amovible à une sonde de phacoémulsification (12), et comprenant :
un orifice d'entrée d'aspiration (66-1) et un orifice de sortie d'aspiration (62-1), l'orifice d'entrée d'aspiration étant conçu pour être accouplé de manière amovible à un canal d'aspiration (47) de la sonde de phacoémulsification, l'orifice de sortie d'aspiration étant conçu pour être accouplé à une conduite tubulaire d'aspiration (46) ;
une section de canal d'aspiration raccordant fluidiquement l'orifice d'entrée d'aspiration à l'orifice de sortie d'aspiration, dans lequel la section de canal d'aspiration comprend une première section (47-1) accouplée à l'orifice d'entrée d'aspiration et conçue pour être accouplée à une extrémité distale (13) de la sonde de phacoémulsification, dans lequel la section de canal d'aspiration comprend une seconde section (47-2) accouplée à l'orifice de sortie d'aspiration ;
un orifice d'entrée d'irrigation (62-2) et un orifice de sortie d'irrigation (66-2), l'orifice d'entrée d'irrigation étant conçu pour être accouplé à une conduite tubulaire d'irrigation (43), l'orifice de sortie d'irrigation étant conçu pour être accouplé de manière amovible à un canal d'irrigation (45) de la sonde de phacoémulsification ;
une section de canal d'irrigation raccordant fluidiquement l'orifice d'entrée d'irrigation à l'orifice de sortie d'irrigation ;
une valve (64) disposée dans la section de canal d'aspiration et conçue pour commander sélectivement une connectivité de fluide dans la section de canal d'aspiration entre l'orifice d'entrée d'aspiration et l'orifice de sortie d'aspiration ; et
un canal de dérivation (52) conçu pour permettre à une partie du fluide d'irrigation dans la section de canal d'irrigation d'entrer dans la section de canal d'aspiration ;
**caractérisé en ce que** le canal de dérivation fournit un raccordement fluidique permanent entre la section de canal d'irrigation et la seconde section de la section de canal d'aspiration.

13. Appareil selon la revendication 12, dans lequel le canal de dérivation raccorde fluidiquement la section de canal d'irrigation à la section de canal d'aspiration au niveau d'une région (47-2) entre la valve et l'orifice de sortie d'aspiration.
